# EUROPEAN PATENT APPLICATION

(11) **EP 4 679 435 A2**
(43) Date of publication of application: **14.01.2026**
(21) Application number: 25182483.5
(22) Date of filing: 12.06.2025
(51) Int. Cl.: G16C 20/80, G16C 20/30, G16C 20/50, G16C 20/70

(54) **SOFTWARE PLATFORM FOR DRUG DISCOVERY**

(30) Priority: 20.06.2024 US 202463662140 P
(71) Applicant: Isomorphic Labs Limited, London EC2M 4RB (GB)
(72) Inventor: Cheung, Jonathan Wai-chuen, London, EC2M 4RB (GB); Murtagh, Robert James, London, EC2M 4RB (GB); Kay, William Thomas, London, EC2M 4RB (GB); Shafi, Amber, London, EC2M 4RB (GB)
(74) Representative: Marks & Clerk GST

(57) **Abstract**

Methods, systems, and apparatus, including computer programs encoded on a computer storage medium, for dynamically updating the display of the user device as a user interacts with the graphical user interface. In one aspect, a method comprises, at each of a plurality of update iterations: initiating the update iteration in response to determining that a user of the user device has interacted with the graphical user interface to modify the chemical structure of a molecule being presented on the display of the user device; automatically determining a respective updated value, for the updated molecule, of each molecule property in the set of molecule properties using the set of updated molecule data; and updating the display of the user device to present: (i) a graphical representation of the updated molecule, and (ii) the respective updated value, for the updated molecule, of each molecule property in the set of molecule properties.

## Description

### BACKGROUND

This specification relates to drug discovery, e.g., the process of identifying new drug compounds for treating a particular disease. In particular, drug discovery can involve target identification, e.g., identifying a specific biological target that is a hallmark of a disease process, and screening a library of chemical compounds to identify molecules that can potentially interact with the biological target as therapeutic agents. Drug discovery can also involve hit optimization, e.g., optimizing identified therapeutic agents to increase their effectiveness, e.g., their absorption, potency, selectivity, etc., while decreasing their toxicity in a biological system.

This specification also relates to user interfaces. User interfaces (UI) can be used to display and interact with data maintained by website infrastructure, including servers, networks, and data storage devices. In particular, website infrastructure can support the uploading, maintaining, and editing of content on a website using a UI configured with a data processing system that can store and access the appropriate data.

### SUMMARY

This specification describes an in-silico drug design system implemented as computer programs on one or more computers in one or more locations that provides computational tools for drug discovery. In particular, the in-silico drug design system can maintain data pertaining to drug discovery projects, e.g., where each project is oriented around a specific user hypothesis for a particular biological target, and one or more models that can process data from a user environment to generate respective outputs that can facilitate the drug discovery process.

The system can expose the one or more models to a user of the in-silico drug design system using a graphical user interface (GUI). More specifically, the system can be configured to process a user input entered by way of the GUI using one or more models in order to generate data, e.g., results from processing the user input using a model, that can be displayed on the GUI for the user. As an example, the user can navigate multiple displays of the GUI to facilitate exploring a specific hypothesis for a particular disease.

Aspects of the invention are set out in independent claims 1 and 24; the subject matter of these claims, and of their dependent claims, may be combined.

Particular embodiments of the subject matter described in this specification can be implemented so as to realize one or more of the following advantages.

The system can streamline the drug discovery process, e.g., from target identification to lead optimization. In particular, the system provides tools for drug discovery that are organized in one convenient location that can be accessible by multiple users. The tools are configured to be used in concert as part of a unified drug discovery project. More specifically, the system facilitates the generation, logging, and collection of data generated as part of the drug discovery process, which reduces the amount of time and effort a user needs to dedicate to manage the drug discovery project.

The system can provide a user interface that allows a user to modify the structure of a candidate molecule in real-time. In particular, the system can provide a user interface that automatically updates in response to a user modification of a chemical structure to display corresponding predicted molecule properties in real-time. The low latency of the update can allow users to rapidly prototype and experiment with candidate molecules at each of a number of modification iterations, thereby facilitating the drug discovery process.

Additionally, the system can automatically maintain project data and save generated outputs for future use, e.g., to prevent the need to perform the same computations more than once. In particular, the system can save computational resources by determining whether or not the required data for an output is precomputed, e.g., stored in a database, before processing an input to perform the same calculation. For example, the system can provide a user interface for automatically determining molecule properties from a modified chemical structure that can be updated relatively quickly by leveraging precomputed values, e.g., within one second of receiving indication of a user modification to the chemical structure, and requires less computational resources relative to processing the updated molecule data using a predictive model to regenerate the molecule properties.

Furthermore, the system provides a novel mechanism for specifying user tolerances in a multiparameter optimization, e.g., a hit optimization for a collection of molecules. In particular, the system allows a user to select intervals and associated desirability ratings for finer-grain control over penalties associated with molecule properties. In this case, the desirability ratings can allow a user to define a spectrum of preference from most preferable to least preferable with respect to possible values of a particular molecule property, which reduces the computational resources necessary to generate the desired results. In particular, rather than running multiple iterations of a process involving repeatedly scoring and filtering molecules according to different molecule property criteria, checking the results for each to see if the identified molecules are acceptable, and updating scoring and filtering parameters for a next iteration, the system can enable a user to specify multiple intervals of molecule property values associated with different desirability ratings.

Scoring and filtering a set of molecules in accordance with the desirability ratings of the multiple different intervals, as enabled by the user interface described in this specification, can replace multiple iterations of scoring and filtering (which may be necessary when using conventional systems) with a single round of scoring and filtering in accordance with the specified desirability ratings of the different intervals. The system described in this specification thus provides an improved user interface that enables a more flexible and descriptive specification of the desirability of various ranges of molecule property values (e.g., as part of scoring and filtering a set of molecules) while reducing consumption of computational resources (e.g., memory and computing power).

The details of one or more embodiments of the subject matter of this specification are set forth in the accompanying drawings and the description below. Other features, aspects, and advantages of the subject matter will become apparent from the description, the drawings, and the claims.

### BRIEF DESCRIPTION OF THE DRAWINGS

FIG. 1 is a system diagram of an example in-silico drug design system.
FIGS. 2A and 2B illustrate an example user interface display for modifying a molecule and viewing associated drug properties of the modified molecule.
FIG. 3 illustrates an example user interface display for selecting intervals associated with desirability ratings that can inform an optimization performed using a collection of molecules.
FIG. 4 illustrates an example user interface display for organizing collected project metadata.
FIG. 5 is an example process for updating a display to present automatically-determined molecule properties for a modified molecule.
FIG. 6 is an example process for determining a loss based on selected intervals and associated desirability ratings.

Like reference numbers and designations in the various drawings indicate like elements.

### DETAILED DESCRIPTION

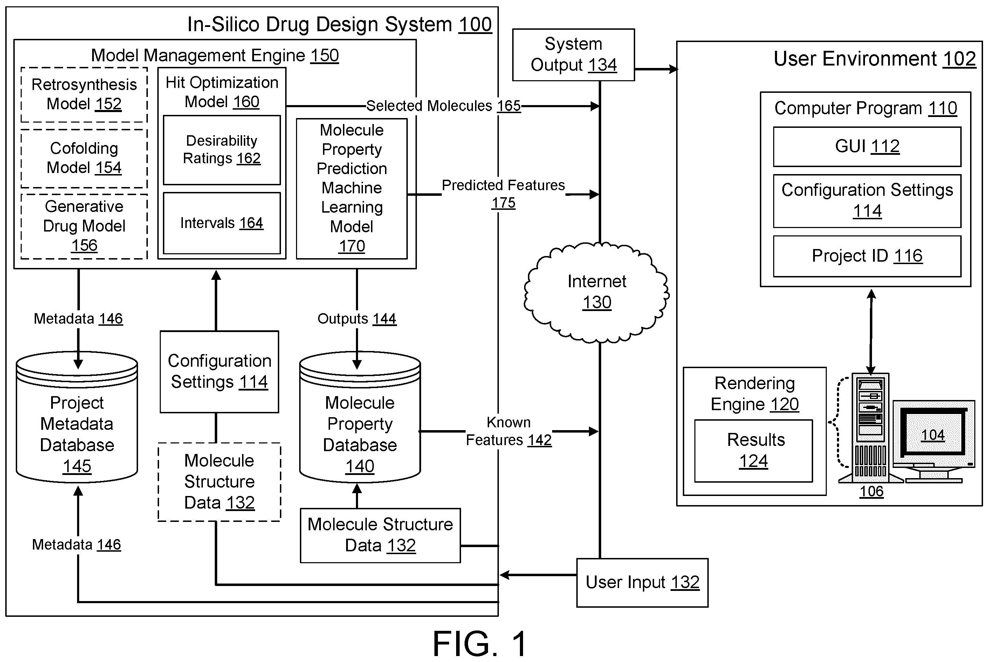

FIG. 1 shows an example in-silico drug design system 100. The in-silico drug design system 100 is an example of a system implemented as computer programs on one or more computers in one or more locations in which the systems, components, and techniques described below are implemented. In particular, the system 100 can access and generate data that can be rendered on a computer display within a user environment 102.

In particular, a computing device 106 presents on a display 104 a graphical user interface (GUI) 112 that allows a user, e.g., the user of computing device 106, to interact with a provided data source. In this case, the provided data source is a computer program 110 generated by the in-silico drug design system 100 to specify the rendering of a GUI 112 of the in-silico drug design system 100, e.g., a GUI for a computational tool that a user can use as part of the drug discovery process. More specifically, the system 100 can generate a computer program 100 that specifies the configuration of a GUI 112 that can be rendered using a rendering engine 120.

In particular, the computer program 110 can contain configuration settings 114, e.g., a set of parameters that control various aspects of the representation of the GUI 112 on the display 104, a set of parameters that control various aspects of the system 100 processing of user-inputted data using the GUI 112, or both, that can be employed by the computing device 106, e.g., using the rendering engine 120, to display the particular configuration of the GUI 112 specified by the configuration settings 114. In particular, the configuration settings 114 can be specific to a particular task a user is executing with the system 100. For example, the configuration settings 114 the system 100 provides for hit optimization can be different than the settings 114 the system 100 provides for target identification, since the GUI 112 the system 100 provides for hit optimization can be different than the GUI 112 the system 100 provides for target identification.

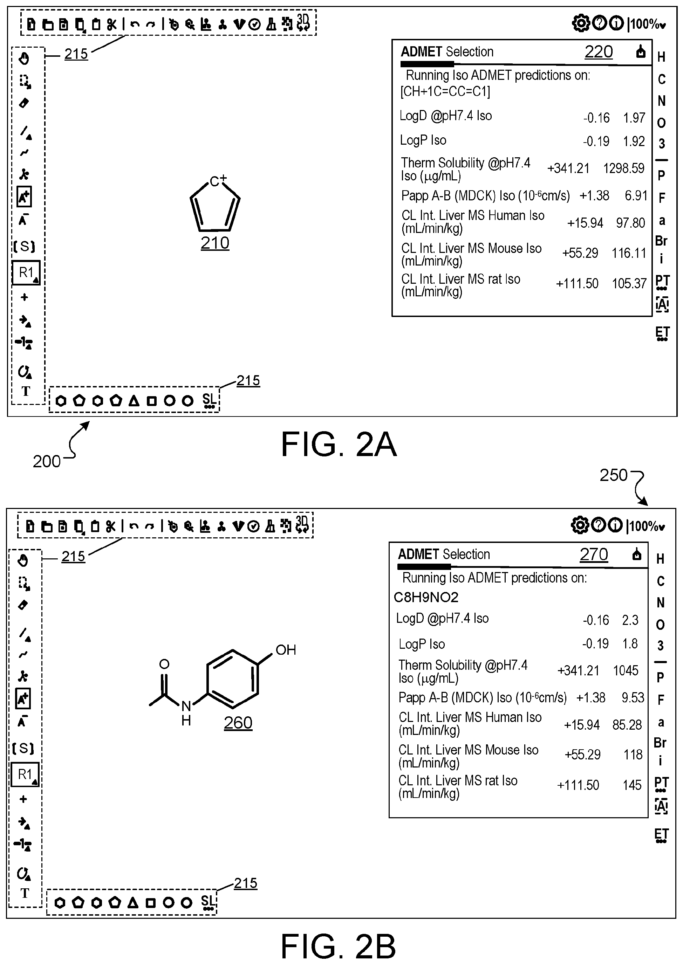

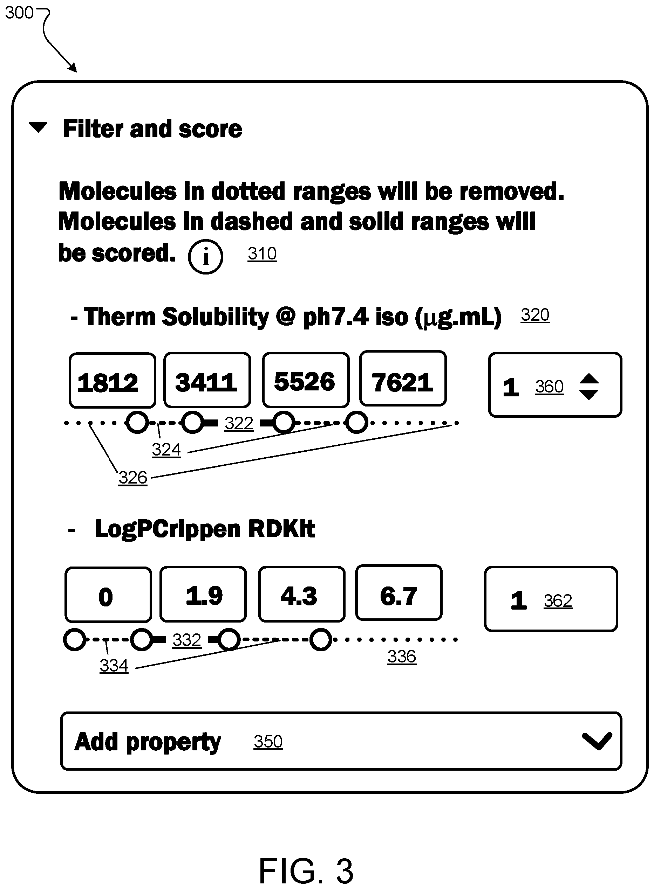

As an example, the system 100 can provide a default set of configuration setting parameters as the configuration settings 114. In some cases, a user can modify the configuration settings 114, e.g., in accordance with one or more user preferences for the GUI 112. Example configuration settings for respective GUIs will be covered with respect to FIGS. 2 and 3. In other cases, the default set of configuration setting parameters is not editable by a user.

In particular, the GUI 112 can include a display of results 124, e.g., results from processing user-entered input data by way of the GUI 112 using the in-silico drug design system 100. As an example, the rendering engine 120 can render a GUI 112 that includes a graphical representation of a molecule with a set of corresponding molecule properties, e.g., as will be described with respect to FIG. 2. As another example, the rendering engine 120 can render a display for configuring a multi-parameter optimization for selecting one or more target drug candidates as hit optimization, e.g., as will be described with respect to FIG. 3.

The GUI 112 can allow the user to provide data to the in-silico drug design system 100, e.g., the user input 132, in response to user interaction with the GUI 112. For example, the GUI 112 can include an input portion, e.g., one or more data fields, for entering data into the system 100. As another example, the GUI 112 can include a graphical representation that can be modified by a user, e.g., by drag-and-dropping, sliding an indicator element along the track of a slider, by drawing a user input, etc. As yet another example, the GUI 112 can include a data upload mechanism, e.g., for a user to provide one or more data files, e.g., molecular data files, for upload to the in-silico drug design system 100.

The in-silico drug design system 100 can receive the user input 132 using a network, e.g., the internet 130 or an intranet. In this case, the system 100 can receive a user input 132 from the user environment 102 and provide a system output 134, e.g., the executable computer program 110 and results 124 of a data processing calculation, using a network, e.g., the internet 130. In the particular example depicted, the system 100 can receive one or more of molecule structure data 132, configuration settings 114, e.g., the subset of the configuration settings 114 that pertains to the processing of user-inputted data, and a project identifier, e.g., the project ID 116. In particular, the system can use the project ID 116 to maintain and organize metadata generated by one or more users through the execution of a project, as will be described in more detail below.

The in-silico drug design system 105 can contain one or more processing engines configured to generate outputs for the user environment 102 and one or more databases configured to maintain data for the user environment 102. In the particular example depicted, the data processing system 105 includes a molecule property database 140, a project metadata database 145, and a model management engine 150. The model management engine 150 can, e.g., maintain one or more models and or route a user input 132 for appropriate processing by the appropriate model.

The molecule property database 140 can maintain data pertaining to one or more molecules that have been previously processed by the system 100. In particular, the database 140 can include a schema that maintains one or more data structures for each molecule or one or more data structures for different molecular families, e.g., organic compound families, protein families, nucleic acid families, etc. As an example, the data structures can be tables, tree-based structures, graphs, or arrays. In some cases, the in-silico drug design system 100 can have received and processed one or more molecular libraries in order to populate the database 140. One example of such a database is ChEMBL maintained by the European Bioinformatics Institute, of the European Molecular Biology Laboratory (Mendez et al., "ChEMBL: towards direct deposition of bioassay data", Nucl. Acids Res, 47, D930-40).

As an example, the data maintained in the database 140 can include data representing the structure of a molecule, e.g., 2D or 3D molecular structure, ADMET (absorption, distribution, metabolism, excretion, and toxicity) properties, etc. As yet another example, the system 100 can collect data generated by processing the user input 132 using the model management engine 150, e.g., the outputs 144 of one or more of the models managed by the model management engine 150 can be stored as part of the database 140. In particular, the system 100 can store a record of all previous generations of model outputs 144, e.g., to allow the system 100 to perform a retrieval operation to identify whether or not the data required as specified by the user input 132 for processing already exists in the database 140.

The project metadata database 145 can maintain and organize metadata 146 collected as part of a project. In this case, a project is a container for organizing different drug discovery tasks executed using the system 100. For example, a project can be directed toward a particular disease and each workspace in the project can relate to different aspects of drug discovery with respect to the disease, e.g., target identification, hit discovery, hit-to-lead optimization, etc. As another example, a project can be directed toward a biological target and each workspace in the project can relate to different aspects of drug discovery with respect to the biological target, e.g., lead optimization.

In particular, the project metadata database 145 can include a schema that maintains one or more data structures for each project, e.g., organized by project ID 116. In the particular example depicted, the system 100 can collect metadata from the user environment 102 and store the data in the proper data structure using the project ID 116 that is included in the computer program 110. In some cases, projects can be collaborative, e.g., multiple users can access the metadata 146 stored in the database 145 and contribute to the same project using the project ID 116, e.g., from corresponding multiple user environments. In this case, each user can access the same project in a respective workspace in their respective user environment 102, e.g., by loading the corresponding metadata for the project from the database 145 as part of the computer program 110 using the project ID 116.

In particular, the project metadata database 145 can maintain a project log that can be accessed by multiple users. As an example, the metadata 146 can include user information that can be derived from a specific user input 132, e.g., user identification information and timestamps of user interaction with the computer program 110. As another example, the system 100 can collect data that pertains to the sequence of tasks that a user has undertaken as part of the project, e.g., data that captures the scientific rationale being explored based on an ordering of the user's interaction with the computer program 110. As yet another example, the system 100 can collect metadata 146 generated by processing the user input 132 using the model management engine 150, e.g., data including the configuration settings 116 used for generating a particular output.

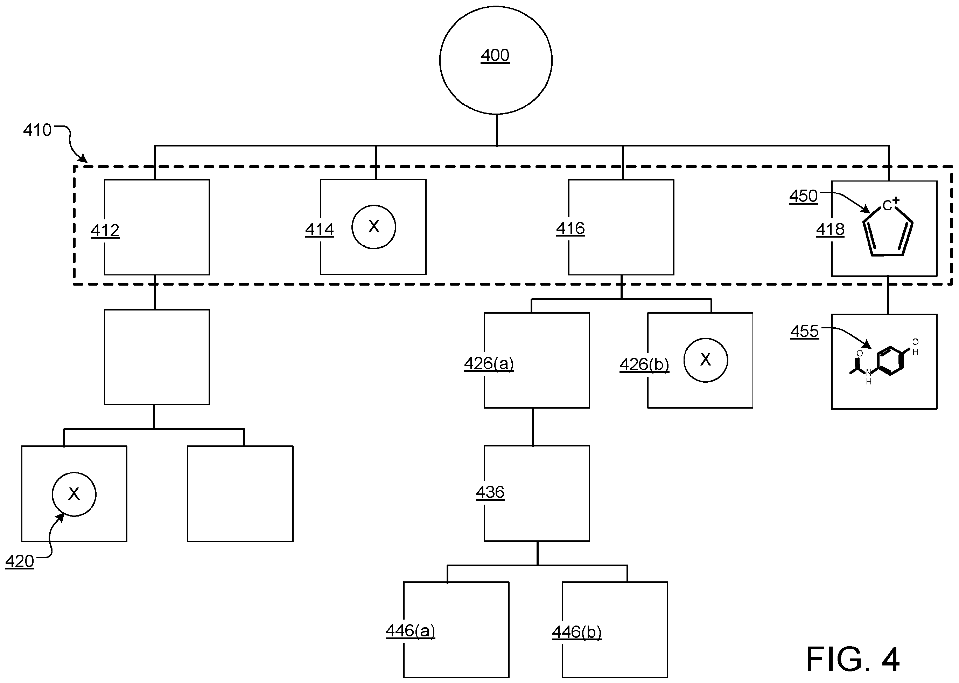

In some cases, the system 100 can configure a GUI 112 for viewing project metadata. For example, the system 100 can present a tree structure that organizes a timeline of the project, e.g., in which each node of the tree can include a graphical representation of an experiment that was run as part of the project, e.g., run using the computer program 110. An example tree visualization will be described in more detail with respect to FIG. 4.

The model management engine 150 can maintain one or more models, each configured to perform a specific drug discovery task. For example, the model management engine 150 can maintain a hit optimization model 160, e.g., a model that can process a number of drug compounds and perform a multi-parameter optimization to select a set of candidate drug compounds, e.g., as will be described in an example below. As another example, the system 150 can maintain a molecule property prediction machine learning model 170, e.g., a model that can process data representing a molecule structure to generate a predicted feature value, e.g., as will be described in an example below. As a further example, the model management engine 150 can optionally maintain a retrosynthesis model 152, e.g., to predict a retrosynthesis pathway of a candidate drug compound; an example is Sun et al. "Energy-based View of Retrosynthesis", arXiv:2007.13437, 2021. The model management engine 150 can optionally maintain a co-folding model 154, e.g., to predict a binding interaction of a protein-ligand complex; an example is AlphaFold3 (Jumper et al., "Accurate structure prediction of biomolecular interactions with AlphaFold 3", Nature 630, 493-500, 2024). The model management engine 150 can optionally maintain a generative drug model 156, e.g., to generate a predicted drug compound that can interact with a target of a particular disease.

In general the in-silico drug design system 100 can be used for obtaining a ligand, in particular a drug, that binds to a target molecule, in particular a drug target such as a protein. For example the target molecule can includes a receptor or enzyme and the ligand can be an agonist or antagonist of the receptor or enzyme. The ligand can be a small molecule (<1000Da) or a biological molecule such as a polypeptide or protein, polynucleotide, or polynucleoside. As another example, the ligand, e.g. the agonist or antagonist, may comprise an antibody or aptamer and the target molecule, e.g. the receptor, may comprise an antibody or aptamer target such as a virus coat protein, or a protein expressed on a cancer cell. For example, the antibody or aptamer may bind to the target and act as an agonist for a particular receptor; alternatively, the antibody or aptamer may prevent binding of another ligand to the target, and hence prevent activation of a relevant biological pathway.

The system can be used for drug discovery by identifying and or evaluating one or more candidate ligands and or drug targets. Identifying a drug can involve using the system to obtain a ligand that functionally interacts with one or more target molecules (a drug may be effective against multiple drug targets). Also or instead the system can be used to screen ligands for off-target effects. In some implementations the system is used for drug discovery by using the system for so-called hit evaluation, e.g. by screening or refining multiple possible drug molecules (candidate ligands) to identify one or more that is particularly potent, selective, or non-toxic, e.g. according to predicted ADMET properties. A potential drug molecule identified or refined by the system can then be selected for physical synthesis. This can involve the user interacting with the system to evaluate ease of physical synthesis of a candidate ligand, using the retrosynthesis model 152. The ligand may be physically synthesized and then, e.g., tested in vitro or in vivo.

In implementations, and as described in more detail below, a current internal state of the in-silico drug design system 100 is provided to the user via the GUI 112. This current internal state comprises a representation of an updated version of a (current) molecule and its predicted properties, and this technical information is used by the user to control the system 100 to guide the system towards designing a drug, in particular for physical synthesis. As described later, the information provided to the user can also include information identifying electrostatic clashes, such as steric hindrance information, and/or information indicating water affinity sites on the updated molecule, e.g. on a ligand that is a potential drug molecule.

In implementations, and as described in more detail below, the current internal state of the in-silico drug design system 100 can be updated the user using the GUI 112. This can involve modifying the chemical structure of the molecule in order to obtain the updated molecule and its predicted properties. In some implementations a user input directly specifies the structure of the updated molecule; in some implementations the user input is used to control the in-silico drug design system 100 to generate a new structure for the updated molecule. More specifically the user input mechanism, in particular the GUI 112, enables the user to generate a structure for the updated molecule by selecting a portion of the (current) molecule to be replaced and then using a generative machine learning model, e.g. the generative drug model 156, to generate a structure for the updated molecule in which the selected portion of the molecule has been generated by the generative machine learning model. The molecule properties of the updated molecule can then be predicted as described elsewhere herein.

In some implementations the GUI 112 can provide a mechanism for multiparameter optimization of a candidate drug molecule. Broadly this can be done by partitioning a range of possible values of one or more molecule properties into a plurality of intervals, and associating each of the plurality of intervals with a respective desirability rating from a set of desirability ratings. This approach can enable the user to set multiple different user tolerances for different respective molecule properties, facilitating optimizing the updated molecule against the set of tolerances (which can otherwise be very difficult and computationally intensive). Implementations of this technique are described further later.

Referring again to FIG. 1, one or more of the models can be machine learning models. In this case, the models can have any appropriate machine learning architecture, e.g., the models can be implemented in part or in whole as a neural network. In the case that one of the models maintained by the model management engine 150 includes a neural network, the neural network can include any appropriate number of neural network layers (e.g., 1 layer, 5 layers, or 10 layers) of any appropriate type (e.g., fully connected layers, attention layers, convolutional layers, etc.) connected in any appropriate configuration (e.g., as a linear sequence of layers or as a directed graph of layers). As another example, one or more of the models can be a random forest model, decision tree model, support vector machine model, linear regression model, etc.

In some implementations the model management engine 150 maintains the models 150 by training the models, e.g., the models 152, 154, 156, and 170, at each of a number of training iterations until a training termination criterion is met. In the case that one or more of the models is a neural network, the model can be trained by calculating and backpropagating gradients of an objective function to update parameter values of the model, e.g., using the update rule of any appropriate gradient descent optimization algorithm, e.g., RMSprop or Adam.

As an example, the engine 150 can start a training process including a number of training iterations for one or more of the models in response to a request from a user. As another example, the engine 150 can start a training process after a specified amount of time has elapsed between the current time and the completion of the most recent training process. In the case in which the user can configure various aspects of the processing of the user input 132 by updating the set of configuration parameters in the configuration settings 114, the user can specify hyperparameters that pertain to the training or the architecture of the models.

In some cases, the model management engine 150 can be additionally configured to access one or more scripts, e.g., executable code or instructions. For example, the engine 150 can access and run user-provided scripts. In this case, the user input 132 can include one or more scripts that can be run by the engine 150 as part of the computer program 110. In particular, the system 100 can provide a GUI 112 as part of a computer program 110 that allows a user to upload, edit, and run an uploaded script to generate an output that is displayed as results 124 by the rendering engine 120 on the computing device 106.

In an example interaction, the user input 132 can contain data defining a modification to the chemical structure of a molecule presented on the display 104 of the computing device 106. An example user interface display for modifying a molecule and viewing associated drug properties of the modified molecule will be described in more detail in FIG. 2. In this case, the in-silico drug design system 100 can process the modified chemical structure, e.g., the molecule structure 132, and determine the corresponding associated drug properties for the user display. More specifically, the system 100 can identify whether the molecule has been previously processed using the molecule property database 140, e.g., in the case that the structure 132 was previously processed by the system 100 as part of a user input 132 or in the case that the structure 132 was included in a library of molecules previously processed by the system. By determining whether or not the required data as part of the system output 134 is precomputed in the molecule property database 140, the system 100 can reduce the computational resources necessary to provide the output 134.

In particular, the system 100 can perform a retrieval operation using the molecule property database 140 to determine if the molecule 132 is indexed in the database 140 with the required data, e.g., that the required data is precomputed and stored in the database 140. As an example, the system can assign a molecular identifier to each molecule structure 132, e.g., such that the molecular identifier can be used as a primary key in a retrieval operation to locate the molecular identifier using the database 140. In the case that the system 100 identifies the molecule structure 132 in the molecule property database 140, the system 100 can return the known features 142 as a system output 134 to the user environment 102. The known features 142 can then be displayed as results 124 as part of the GUI 112, e.g., using the rendering engine 120, as will be described in more detail with respect to FIG. 2.

In the case that the system 100 does not identify the molecule structure 132 in the molecule property database 140, e.g., the molecule 132 is not indexed in the database, the system 100 can initialize a new row or table in the database 140, e.g., according to the schema being used to store the data, for the molecule structure 132. The system 100 can then process the molecule structure data 132 using the model management engine 150 to generate the features. In this case, the model management engine 150 can route the molecule structure data 132 as input to the molecule property prediction machine learning model 170.

In particular, the model 170 can be a machine learning model that is configured to process data representing a molecular structure to predict one or more molecule features in a set of molecule properties, e.g., the predicted features 175, corresponding with the molecule structure 132. As an example, the set of molecule properties can include one or more ADMET properties, atomic features, structural features, topological features, physical and chemical properties, etc. The predicted features 175 can be provided to the user environment 102, e.g., using the internet 130, as part of the system output 134 and displayed as results 124 using the GUI 112. There are many models available that can be used for molecule property prediction. For example, models and tools for ADMET prediction are available in the DeepChem library ("Deep Learning for the Life Sciences", Ramsundar et al., 2019); and RDKit (Landrum, "RDKit: Open-source cheminformatics", 2013; rdkit.org) can be used for predicting various molecule properties including physicochemical properties.

In another example interaction, the user input 132 can contain configuration settings 114 for the hit optimization model 160. In this case, the hit optimization model 160 can be configured to perform a multi-parameter optimization to identify a set of candidate molecules, e.g., drug compounds, from a collection of molecules based on a desirability rating for a set of molecule properties. In particular, the configuration settings 114 can include a subset of property-specific desirability ratings 162 corresponding with user-defined intervals 164 of possible values for each molecule property included in the multi-parameter optimization. More specifically, the system 100 can receive data that partitions a range of possible values of a molecule property into intervals and associates each of the intervals with a respective desirability rating from a set of desirability ratings. The desirability ratings can allow for finer-grain user control over hit optimization, e.g., the desirability ratings 162 can allow a user to define a spectrum of preference from most preferable to least preferable with respect to possible values of a particular molecule property in the multi-parameter optimization. An example display that provides a slider for selecting intervals associated with desirability ratings that can inform an optimization performed for a library of molecules, e.g., by adjusting indicator elements that correspond with the respective endpoints of each interval using the slider, will be described in more detail in FIG. 3.

The system 100 can then process a collection of molecules, e.g., a library of molecules, in accordance with the desirability ratings 162 and intervals 164 using the hit optimization model 160 to identify candidate molecules. In particular, the system 100 can incorporate the desirability ratings 162 and corresponding intervals 164 into the loss function of the hit optimization model 160, e.g., to determine a respective property-specific loss based on a desirability rating of an interval that includes the value of the molecule property for the molecule. For example, the system 100 can use the desirability ratings 162 and intervals 164 to penalize molecules with a property value within the interval with the least preferable desirability rating more than molecules with a property value within the interval with the most preferable desirability rating. The identified candidate molecules can then be provided to the user environment 102, e.g., using the internet 130, as part of the system output 134 and displayed as results 124.

FIG. 2 illustrates an example user interface display for modifying a molecule and viewing one or more molecule properties of the modified molecule. In particular, the in-silico drug design system 100 of FIG. 1 can provide the GUI to a computing device to render the displays 200 and 250.

FIG. 2A depicts a visualization of a first molecule with current values of a set of molecule properties, and FIG. 2B depicts a visualization of a second molecule, e.g., a structure made by modifying the first drug compound, with respective updated values of the set of molecule properties. Both FIGS. 2A and 2B depict respective values of the set of molecule properties that are provided for illustrative purposes, i.e., the values displayed are not predicted or experimental values provided by the system.

The display 200 illustrates a graphical representation of a first molecule 210, e.g., the molecule [CH+1C=CC=C1], along with corresponding current values of a set of molecule properties 220 for the molecule 210. Likewise, the display 250 illustrates a graphical representation of a second molecule 260, e.g., the molecule C8H9NO2, along with corresponding updated values for the set of molecule properties 270 for an updated molecule 260, i.e. an updated version of a current molecule (which in this example is molecule 210).

In particular, the in-silico drug design system 100 of FIG. 1 can provide the GUI to a computing device to render the displays 200 and 250 using a set of molecule data and a set of updated molecule data, respectively. As an example, the set of molecule data can be a simplified molecular-input line-entry system (SMILES) string that represents the structure of the molecule. As another example, the set of molecule data can be a molecular fingerprint of the molecule, e.g. a numerical representation that characterizes the molecule, in particular structural features of the molecule. As yet another example, the set of molecule data can be sourced from a structural data file, e.g., protein databank files, structure data files, chemical markup files, etc.

In some cases, the current molecule, e.g., the first molecule 210, can be selected from a molecule database, e.g., where each molecule is associated with a respective set of molecule data defining the chemical structure. In this case, the system 100 can maintain a repository of molecules and structures, e.g., from processing one or more molecule libraries. As an example, the system 100 can receive a request to access the set of molecule data for the current molecule from the molecule database for rendering, e.g., on the display 200.

In the particular example depicted, the graphical representations of the chemical structure of the molecules 210 and 260 are two-dimensional (2D) skeletal representations. As another example, the graphical representations of the chemical structure can be a 2D Lewis or ball-and-stick molecule representation. As yet another example, the graphical representation of the chemical structure can be a three-dimensional (3D) molecular representation. In the case that the graphical representation is a 3D molecular representation, the system can allow the user to dynamically reorient the molecule, e.g., by drag-and-dropping, to provide different views of the chemical structure on the display.

In this case, the set of molecule properties 220 and 270 include absorption, distribution, metabolism, excretion, and toxicity properties. As an example, the set of molecule properties 220 and 270 can include a partition coefficient (LogP), e.g., a measure of the lipophilicity of the rendered molecule, e.g., an indicator of a molecule's ability to be absorbed in a lipid-containing system, e.g., a biological system. As another example, the set of molecule properties 220 and 270 can include a distribution coefficient (LogD), e.g., a measure of the lipophilicity of the rendered molecule at different acidities (pH). As yet another example, the set of molecule properties 220 and 270 can include a thermosolubility and liver metabolism rates in one or more animal models, e.g., a human, mouse, and rat model.

In some cases, the system 100 can allow a user to configure the displays 200 and 210, e.g., using one or more of the configuration parameters of the configuration settings 114 of FIG. 1. In particular, the system 100 can allow the user to configure default settings for the user's desired presentation of the rendered molecule on the displays 200 and 250. As an example, the one or more configuration parameters can specify colors for rendering the chemical structure, e.g., different colors for rendering different portions of the chemical structure, the opacity of the chemical structure, etc. As another example, the one or more configuration parameters can specify whether additional annotations, e.g., a marking denoting electrostatic clashes (e.g. steric hindrance) or the display of water molecules where they would bind with respect to hydrophilic parts of the molecule, are rendered as part of the graphical representation. In the case of displaying water molecules, the system 100 can run a water-mapping model to determine the placement of the water molecules on the molecule. There are several examples of such models; one is WATsite (Yang et al., WATsite2, Journal of Chemical Information and Modeling, 54(10), 2987-2995, 2014).

The system 100 can dynamically update the display of the graphical representation of the chemical structure of the molecule as a user interacts with the GUI. In particular, the system 100 can receive a user input defining a request to modify the chemical structure of the molecule being presented on the display, e.g., the GUI can be interactive. As an example, the system can provide one or more options for modifying the molecule, e.g., the options 215.

For example, the system 100 can determine that a user has drawn an addition to or deleted part of the graphical representation of the chemical structure, e.g., by drawing or erasing part of the graphical representation of the chemical structure on the GUI. As another example, the system 100 can determine that a user has selected and rotated a portion of the molecule around a rotatable bond, e.g., by drag-and-dropping a portion of the molecule. As yet another example, the system 100 can determine that a user has input data specifying a modification to the chemical structure into an input portion, e.g., an input portion configured for the input of one or more alphanumerical characters. In some cases, the input portion can receive a prompt indicating an instruction for modification. As a further example, the system 100 can determine that a user has selected and replaced a fragment of a molecule, e.g., by selecting a replacement fragment from an accessible predefined collection of molecule fragments. In this case, the system 100 can provide the predefined collection of molecule fragments to the user upon an indication that the user intends to replace a fragment of a molecule.

In response to a request to update the graphical representation of the chemical structure, e.g., the user modifying the molecule 210 into the molecule 260, the system 100 can update the display of the graphical representation. In particular, the system 100 can update the graphical representation based on an updated set of molecule data in accordance with the updated molecule structure. As an example, the system 100 can modify a SMILES string representing the molecule using a rules-based system to adjust the data according to the chemical structure modification received by the system 100. For example, the system 100 can use a generative machine learning model, such as the generative drug model 156, to generate the set of updated molecule data based on the modification to the chemical structure of the molecule. In some cases, the system 100 can condition the generative machine learning model on data identifying the portion of the molecule that was modified by the user interaction, e.g., the system can identify the scaffold that retains the same structure and the portion of the molecule to be replaced. There are various such models available; one example for small molecules is Reinvent (Loeffler et al., "Reinvent 4: Modern AI-driven generative molecule design", Journal of Cheminformatics v. 16, 2024); an example for biomolecules is RFdiffusion (Watson et al. "De novo design of protein structure and function with RFdiffusion", Nature, 2023, 620, 1089-1100).

The system 100 can then use the updated set of chemical structure data to automatically determine respective updated values for each molecule property in the set of molecule properties. In particular, the system 100 can update the display 200 to the display 250 in response to the modification to the molecule. As an example, the system 100 can update the display with the respective updated values of the updated molecule in real-time, e.g., within .5 seconds, 1 second, 2 seconds, or 10 seconds, of determining that the user of the user device has interacted with the graphical user interface.

In some cases, the system 100 can identify one or more precomputed values of the set of the molecule properties for display. For example, the system 100 can determine that a precomputed value of the set of molecule properties for the updated molecule 260 is available in a molecule property database, e.g., the molecule property database 140 of FIG. 1, and can use one or more retrieval operations to identify and select each precomputed value from the database 140, e.g., using an index. In the case that the system 100 determines that a precomputed value of the molecule property is not available in the molecule database, the system can process the updated set of chemical structure data using a molecule property prediction machine learning model, e.g., the molecule property prediction machine learning model 170 of FIG. 1.

In particular, the system 100 can process a model input based on the set of updated molecule data using the molecule property prediction machine learning model 170 to generate the updated value of the molecule property of the updated molecule. The molecule property prediction machine learning model 170 can have any appropriate machine learning architecture, e.g., a neural network, maximum likelihood optimization, a decision tree, a random forest, a support vector machine, or a combination thereof, that can be configured to process an input including the updated set of chemical structure data to generate an updated set of molecule properties.

In the case that the molecule property prediction machine learning model 170 is implemented as a neural network, the molecule property prediction machine learning model 170 can have any appropriate number of neural network layers (e.g., 1 layer, 5 layers, or 10 layers) of any appropriate type (e.g., fully-connected layers, attention layers, convolutional layers, etc.) connected in any appropriate configuration (e.g., as a linear sequence of layers, or as a directed graph of layers). In this case, the molecule property prediction machine learning model 170 can be trained on a set of training examples, e.g., where each training example corresponds to a respective training molecule and includes a training model input and a corresponding target set of molecule properties. In particular, the system can train the molecule property prediction machine learning model 170 to optimize an objective function that measures a discrepancy between the target set of molecule properties and the predicted set of molecule properties, e.g., by updating the respective values of parameters of the model 170, e.g., using the update rule of any appropriate gradient descent optimization algorithm, e.g., RMSprop, or Adam.

In some cases, the model management engine 150 can maintain two molecule property prediction models (not depicted in FIG. 1). In particular, the engine 150 can include a first computationally-lightweight molecule property machine learning model configured to predict the values of the set of molecule properties with lower accuracy and a second, computationally-intensive molecule property machine learning model configured to predict the values of the set of molecule properties with higher accuracy, e.g., the computationally lightweight model can include less model parameters than the computationally-intensive model. As an example, the system 100 can generate the set of predicted molecule properties using the faster lightweight model for display on the graphical user interface, e.g., of the display 250, while simultaneously generating the set of molecule properties using the slower higher-accuracy model. In this case, the system can provide a designation that the displayed results 124 will be updated, e.g., by using a different color to display the predicted values of the faster lightweight model, e.g., in yellow, and the slower-higher-accuracy model, e.g., in green, or marking that the values from the faster lightweight model are preliminary results in some other way.

As an example, the system 100 can then store the set of updated molecule data and the updated value of the molecule property in the molecule property database 140, e.g., as a precomputed value that can be identified based on an index for a future calculation that includes determining the precomputed value. More specifically, the system 100 can maintain precomputed values in the molecule property database 140 with corresponding molecule identifiers. In particular, the system 100 can determine that a precomputed value, e.g., that was previously generated by the molecule property prediction machine learning model 170, is available in the molecule database and return the precomputed value to the user instead of recalculating the value of a feature for a chemical structure that has already been processed by the system 100.

FIG. 3 illustrates an example user interface display for selecting intervals associated with desirability ratings for one or more molecule properties that can inform an optimization performed for a collection of molecules. In particular, the in-silico drug design system 100 of FIG. 1 can provide the GUI to a computing device to render the display 300 as part of a user flow for configuring a multi-parameter optimization.

In particular, the system 100 can use the display 300 for configuring a multi-parameter search optimization to identify one or more molecules with certain desirable properties as part of a hit optimization on the collection of molecules. In this case, the system 100 can identify hit compounds that meet certain criteria, e.g., as specified by the user using the desirability ratings and intervals. As an example, the system 100 can provide the display 300 for a user to configure user preferences with respect to one or more criteria for the multi-parameter optimization.

More specifically, the display 300 can allow the user to select intervals of values with corresponding desirability ratings for each molecule property, e.g., the intervals 164 and associated desirability ratings 162, to provide a user with finer-grain control of the multi-parameter optimization on the collection of molecules. In particular, each molecule property can be associated with one or more desirability ratings, e.g., user tolerances for molecules with values of a particular molecule property in the range specified by the interval. In some cases, the system 100 can require the specification of a minimum number of desirability ratings for each molecule property, e.g., 3, 5, 10, etc.

The system 100 can use the corresponding desirability ratings to evaluate each molecule in the collection of molecules using a loss function that penalizes undesirable properties, e.g., as specified by the desirability ratings. For example, the system 100 can determine a respective value of the molecule property for the molecule, e.g., by processing a model input that characterizes the molecule using the property prediction machine learning model 170, and can determine a respective property-specific loss based on the desirability rating of an interval that includes the value of the molecule property for the molecule. The system 100 can then generate a loss score for the molecule by combining the respective property-specific losses for each of the one or more molecule properties, e.g., as an aggregation (e.g., summation) of particular property-specific losses based on the values of the respective property with respect to the desirability ratings and intervals.

More specifically, the system 100 can apply the penalty function, e.g., a loss function, associated with the interval that includes the value of the molecule property to the value of the molecule property. As an example, in the case of a user specifying three desirability ratings for a molecule property, the system 100 can apply a constant zero loss function to the most desirable rating, e.g., to compute no loss for molecules with property values in the most desirable interval, a linear loss function to the second most desirable rating, e.g., to compute a linear loss for molecules with property values in the second most desirable rating, and a quadratic loss function to the third most desirable rating, e.g., to compute a quadratic loss function for molecules with property values in the third most desirable rating.

For each molecule property, the system 100 can allow a user to specify a partitioning of a range of possible values for the molecule property and associate the range with a respective desirability rating, e.g., using a GUI. In the particular example depicted, the GUI includes a slider track that represents the range of possible values for a molecule property. As another example, the system 100 can provide multiple input portions for entering the values of the partitioned ranges.

As an example, the display 300 can include instructions 310 that explain how a user can specify the criteria for a number of molecule properties using the display 300. In this case, the user can add one or more molecule properties by identifying each molecule property from a list using a drop-down menu, e.g., the add property drop-down menu 350. In other cases, the user can add properties using an input portion, e.g., to enter a molecule property in a text field.

In the particular example depicted, the instructions 310 describe that the desirability ratings correspond with different line markings on the slider, e.g., the line markings in between the indicator elements. For example, the instructions 310 specify that molecules in the dotted ranges are deemed the least desirable, molecules in the dashed ranges are deemed acceptable, and molecules in the solid ranges are most acceptable As another example, the system can display different colors between the indicator elements for each desirability rating, e.g., the molecules in the least desirable range, acceptable range, and solid range can be associated with the colors red, yellow, and green, respectively.

In some cases, the system 100 can present the display 300 with one or more default settings, e.g., the system 100 can provide a default setting with a corresponding set of intervals and desirability ratings for each molecule property. In this case, the system 100 can present the display 300 with predefined indicator element positions along the slider track. As an example, the default setting of the interval endpoint values can be machine learned, e.g., using a machine learning model that has been configured to determine respective intervals and desirability ratings based on a distribution of values of the molecule property over a collection of molecules.

As another example, the positions of the indicator elements can be initialized with default settings determined based on an identified protein target. In particular, the system 100 can receive data identifying a target protein and can determine a collection of similar proteins that satisfy one or more similarity criteria with respect to the target protein, e.g., proteins that have at least a threshold level of similarity based on a comparison of the amino acid sequence of each respective similar protein to the amino acid of the target protein. The system 100 can then identify a set of one or more ligands that bind to each of the similar proteins in the collection and can evaluate the distribution of the sets of predicted molecule properties for the ligands, e.g., the system 100 can use each value distribution of the aggregated sets of predicted molecule properties to determine the default intervals. As an example, the system 100 can use the standard deviation or another dispersion statistic to determine the intervals, e.g., the system 100 can determine that the range of values within one standard deviation from the mean is the most acceptable interval, the range of values within two standard deviations is an acceptable interval, and the range of values greater than two standard deviations from the mean is least desirable.

In the particular example depicted, the user can specify the respective intervals for each of the desirability ratings for each molecule property by sliding, e.g., by selecting and dragging, the indicator elements along the slider track to determine the endpoints of the specified intervals. More specifically, the system 100 can receive the data that partitions the range of possible values for the molecule property and associates each of the intervals with a respective desirability rating as a user moves one or more of the indicator elements that define the endpoints of the specified intervals to define an adjustment to the range of values in the interval. In particular, the system 100 can receive an indication that the user has dragged a position of an indicator element along the track using the GUI to adjust the position of the indicator element.

As an example, the user has configured three desirability ratings for the first molecule property 320, e.g., by moving the positions of the indicator elements, to specify the solid range 322 as the preferred interval of values, the dashed range 324 as the acceptable interval of values, and the dotted range 326 as the least acceptable interval of values. In this case, the specification of the acceptable range 324 and least acceptable range 326 includes values that increasingly deviate from the preferred range 322.

As another example, the user has configured three desirability ratings for the second molecule property 330, e.g., by moving the positions of the indicator elements, to specify the solid range 332 as the preferred interval of values, the dashed range 334 as the acceptable interval of values, and the dotted range 336 as the least acceptable range of values. In this case, the specification of the least acceptable range 336 is defined on only one side of the acceptable values 332 and 334.

In some cases, the system 100 can also provide an input portion for specifying a scaling factor for each property-specific loss, e.g., the input portions 360 and 362. For example, the scaling factor can be an importance factor that denotes a significance of the molecule property, e.g., a relative significance with respect to the other molecule properties specified in the multi-parameter optimization. In particular, the system 100 can scale each property-specific loss by an importance factor corresponding to the property-specific loss as part of generating the loss score for the molecule, e.g., the system 100 can scale each property-specific loss before aggregating the property-specific losses to determine the loss score.

In the particular example depicted, the user has elected to not scale the property-specific losses, e.g., by maintaining the scaling parameters in the input portions 360 and 362 for the molecule property 320 and 330, respectively, at a value of one. In the case that the user has elected to scale the property-specific losses, the system can apply the scaling factor to the loss score calculated for the molecule, e.g., by multiplying the scaling factor by the value of the property-specific loss determined from the loss function corresponding with the desirability rating for the property value of the molecule.

FIG. 4 illustrates an example tree visualization user interface display for collected project metadata. The tree visualization can organize the relationships between each experiment run, e.g., as part of a project using the in-silico drug design system 100 of FIG. 1.

In particular, the root node 400 of the tree can represent a first experiment run, and each node of the tree that stems from the root node or another node can respect a parent-child hierarchy in relation to changes that were made with respect to a different experiment. In this case, the first derivative experiments 410 of the first experiment run, as represented by the root node 400, are the child nodes 412, 414, 416, and 418. More specifically, each level of the tree can respect a parent-child hierarchy, e.g., child node 416 represents a first derivative experiment with respect to the first experiment run, child nodes 426(a) and 426(b) represent a second derivative experiment with respect to the first experiment run, child node 436 represents a third derivative experiment with respect to the first experiment run, and child nodes 446(a) and 446(b) represent a fourth derivative experiment with respect to the first experiment run.

In some cases, the GUI 112 of the tree structure can be interactive, e.g., such that a user can select a node of the tree and access the corresponding data, e.g., by loading the metadata pertaining to the experiment of the node into their workspace as part of the computer program 110. For example, the system can provide a representation of the experiment represented by the node, e.g., using a corresponding image, icon, or text, such that a user can identify which node to select to access a desired experiment at the stage indicated by the node.

As an example, one or more of the nodes can contain a representation of the experiment run, e.g., the representations 450 and 455, which depict the example molecule and modified molecule of displays 200 and 250 of FIG. 2, respectively. In this case, the modified molecule 455 is displayed in a child node of the node of the initial molecule 450, e.g., since the modified molecule 455 was derived from modifications made to the initial molecule 450. As another example, an experiment that did not led to any meaningful outcome for further research and development can be represented by a terminal node, e.g., as depicted by the circle with the x inside.

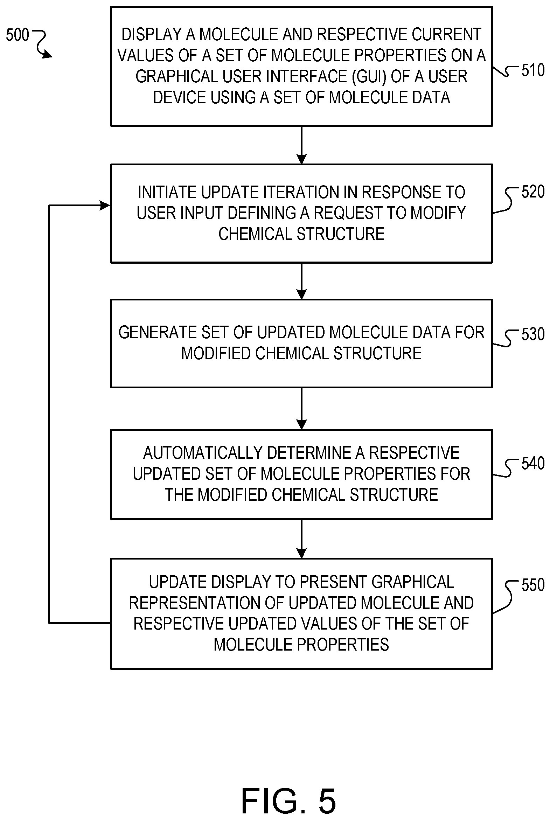

FIG. 5 is a flow diagram of an example process for updating a display to present automatically determined molecule properties for a modified chemical structure. For convenience, the process 500 will be described as being performed by a system of one or more computers located in one or more locations. For example, an in-silico drug design system, e.g., the in-silico drug design system 100 of FIG. 1, appropriately programmed in accordance with this specification, can perform the process 500.

The system can display a molecule and respective values of a set of molecule properties, e.g., a set of one or more of absorption, distribution, metabolism, excretion, and toxicity properties, on a graphical user interface (GUI) of a user device using a set of molecular data (step 510). In particular, the system can display a graphical two-dimensional, e.g., skeletal, Lewis, ball-and-stick, etc. or three-dimensional representation of the chemical structure on the GUI. In the case that the system displays a three-dimensional representation, the system can configure the GUI to reorient the molecule representation in response to a user selecting a portion of the molecule and dragging the portion along one or more directions. As an example, the system can receive a user input that selects the current set of molecular data from a molecule database that includes chemical structure data for a number of molecules. As another example, the system can receive an upload of a chemical structure data file, e.g., a protein databank file, a structure data file, a molfile, etc.

In some cases, the graphical representation provided by the system is configurable by the user, e.g., by specifying one or more configuration values in accordance with user preferences. As an example, the one or more configuration parameters can specify colors for rendering the chemical structure, e.g., different colors for rendering different portions of the chemical structure, the opacity of the chemical structure, etc. As another example, the one or more configuration parameters can specify whether additional annotations, e.g., a marking denoting electrostatic clashes or the display of water molecules where they would bind with respect to hydrophilic parts of the molecule, are rendered as part of the graphical representation.

The system can initiate an update iteration in response to a user input defining a request to modify the chemical structure (step 520). In particular, the GUI can be interactive. For example, the system can determine that a user has drawn an addition to or deleted part of the graphical representation of the chemical structure, has selected and rotated a portion of the molecule around a rotatable bond, e.g., by drag-and-dropping a portion of the molecule, or has input data specifying a modification to the chemical structure into an input portion. As another example, the system can determine that a user has selected and replaced a fragment of a molecule, e.g., by selecting a replacement fragment from an accessible predefined collection of molecule fragments.

The system can generate a set of updated molecule data for the modified chemical structure (step 530). As an example, the system can update the set of molecule data by making corresponding changes to the data that represents the chemical structure, according to the user input. For example, the system can modify a simplified molecular-input line-entry system (SMILES) string representing the molecule to adjust the data according to the chemical structure modification. As another example, the system can use a generative machine learning model to generate the set of updated molecule data based on the modification to the chemical structure of the molecule. In this case, the system can condition the generative machine learning model on data identifying the portion of the molecule to be replaced.

The system can automatically determine a respective updated set of molecule properties for the modified chemical structure (step 540). For example, the system can determine that a precomputed value of the molecule property of the updated molecule is available in a molecule database, e.g., by determining whether a molecule identifier that identifies the updated molecule is indexed in the molecule property database. In this case, the system can access the precomputed value of the molecule property, e.g., using a molecule identifier as an index. As another example, the system can process the set of updated molecule data using the molecule property prediction machine learning model to generate updated values of the set of molecule properties of the updated molecule. In this case, the system can store the set of updated molecule data and the updated molecule property values in the molecule database, e.g., for retrieval in a future operation.

The system can then update the display to present the graphical representation of the updated molecule and respective updated values of the set of molecule properties, e.g., using the updated set of data (step 550). In particular, the system can update the display of the user device to present the graphical representation of the updated molecule and the respective updated values of each molecule property in the set of molecule properties, e.g., within one second of determining that the user has interacted with the graphical user interface. As an example, the system can repeat the steps 520-550 at each of a number of iterations, e.g., each time a user modifies the molecule being displayed, to allow a user to successively modify the chemical structure of a molecule.

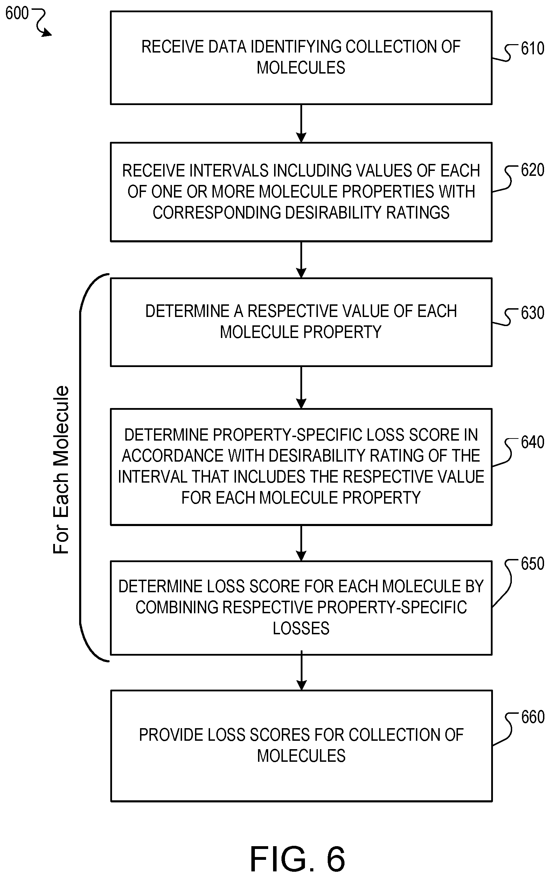

FIG. 6 is an example process for determining a loss based on selected intervals and associated desirability ratings. For convenience, the process 600 will be described as being performed by a system of one or more computers located in one or more locations. For example, an in-silico drug design system, e.g., the in-silico drug design system 100 of FIG. 1, appropriately programmed in accordance with this specification, can perform the process 600.

The system can receive data identifying a collection of molecules (step 610). For example, the collection of molecules can be one or more molecules from a library of molecules. As another example, the system can allow a user to upload a list of identified molecules, e.g., a collection of custom ligands. The system can receive intervals including values of each of one or more molecule properties with corresponding desirability ratings (step 620), e.g., user-specified tolerances for accepting molecules with values in the ranges of values specified by the intervals. In some cases, the system can require that the set of desirability ratings includes at least three desirability ratings. In particular, the system can receive the intervals and corresponding desirability ratings from a graphical user interface (GUI), e.g., a user interface that allows a user to identify respective endpoints for each of the plurality of intervals.

As an example, the system can present a user with a slider interface that allows a user to configure the respective endpoints by sliding one or more indicator endpoints corresponding with the range of values for each interval along a slider track that represents the possible values of a molecule property. In this case the system can receive a user input that identifies the respective endpoints from adjustments to the respective positions of the indicator elements, e.g., based on the user dragging a position of one or more of the indicator elements along the track to adjust the position of the indicator element. In some cases, the system can display the different intervals with different colors according to the desirability rating associated with the interval, e.g., green for most desirable, yellow for moderately desirable, red for least desirable, etc.

As another example, the system can provide multiple input portions for entering the values of the partitioned ranges. In some cases, the system can provide a default setting with a corresponding set of intervals and desirability ratings for each molecule property. In this case, the default setting can be machine learned, e.g., by determining the intervals and corresponding desirability ratings based on a distribution of values of the molecule property over a collection of molecules. In particular, the system can receive data identifying a target protein, determine a collection of similar proteins that satisfy one or more similarity criteria with respect to the target protein, identify a set of ligands, and can determine the distribution of values of the molecule property by docking the ligands with the collection of similar proteins.

The system can then determine a respective value of each molecule property (step 630). As an example, the system can determine the respective value of the molecule property using a machine learning model that has been configured to predict the value of the molecule property. As another example the system can calculate the respective value of the molecule property directly. As yet another example, the system can determine the respective value of the molecule property by identifying a precomputed property value in a molecule property database.

The system can then determine a property-specific loss score in accordance with the corresponding desirability rating of the interval that includes the respective value for each molecule property (step 640), and the system can determine a loss score for each molecule by combining respective property-specific losses (step 650). More specifically, the system can determine the property-specific loss for the molecule property by applying the penalty function associated with the interval that includes the value of the molecule property to the value of the molecule property.

In particular, the system can apply a larger penalty to less desired values, e.g., the system can use the corresponding desirability ratings to determine the appropriate penalization, e.g., loss function type, for the particular property for a particular molecule based on the corresponding desirability rating for the value of the molecule property. As an example, in the case of three desirability ratings, the system can apply a constant zero loss function to the most desirable rating, a linear loss function to the second most desirable rating, and a quadratic loss function to the third most desirable rating. The system can then combine, e.g., aggregate, sum, or average, the respective property-specific losses for each of the molecule properties being evaluated into a loss score. In some cases, the system can also scale the property-specific losses, e.g., using an importance factor, as part of the aggregation.

The system can repeat steps 630-650 until the system has determined a loss score for each molecule in the collection of molecules. The system can then provide the loss scores for the collection of molecules (step 660), e.g., as part of a multi-parameter optimization for identifying a set of candidate molecules from the collection of molecules. As an example, the system can determine a ranking of the molecules based on the loss scores, e.g., as hit compounds. In particular, the system can calculate the losses and take an action according to the loss, e.g., the system can remove molecules with high losses, rank the molecules based on the losses, or both. In implementations the candidate molecules are selected for further evaluation in silico or in the real world. For example one or more of the candidate molecules can be physically synthesized, or selected for synthesis; and can then be evaluated.

In some cases, the system can additionally evaluate a binding affinity for each of the molecules in the collection of molecules and rank the molecules based on the binding affinity. In particular, the system can use the loss scores to select a subset of candidate molecules. The system can then select one or more of the identified set of candidate molecules for physical synthesis based at least in part on the binding affinity.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

Embodiments of the subject matter and the functional operations described in this specification can be implemented in digital electronic circuitry, in tangibly-embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non-transitory storage medium for execution by, or to control the operation of, data processing apparatus. The computer storage medium can be a machine-readable storage device, a machine-readable storage substrate, a random or serial access memory device, or a combination of one or more of them. Alternatively or in addition, the program instructions can be encoded on an artificially-generated propagated signal, e.g., a machine-generated electrical, optical, or electromagnetic signal, that is generated to encode information for transmission to suitable receiver apparatus for execution by a data processing apparatus.

The term "data processing apparatus" refers to data processing hardware and encompasses all kinds of apparatus, devices, and machines for processing data, including by way of example a programmable processor, a computer, or multiple processors or computers. The apparatus can also be, or further include, special purpose logic circuitry, e.g., an FPGA (field programmable gate array) or an ASIC (application-specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub-programs, or portions of code. A computer program can be deployed to be executed on one computer or on multiple computers that are located at one site or distributed across multiple sites and interconnected by a data communication network.

In this specification the term "engine" is used broadly to refer to a software-based system, subsystem, or process that is programmed to perform one or more specific functions. Generally, an engine will be implemented as one or more software modules or components, installed on one or more computers in one or more locations. In some cases, one or more computers will be dedicated to a particular engine; in other cases, multiple engines can be installed and running on the same computer or computers.

The processes and logic flows described in this specification can be performed by one or more programmable computers executing one or more computer programs to perform functions by operating on input data and generating output. The processes and logic flows can also be performed by special purpose logic circuitry, e.g., an FPGA or an ASIC, or by a combination of special purpose logic circuitry and one or more programmed computers.

Computers suitable for the execution of a computer program can be based on general or special purpose microprocessors or both, or any other kind of central processing unit. Generally, a central processing unit will receive instructions and data from a read-only memory or a random access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser. Also, a computer can interact with a user by sending text messages or other forms of message to a personal device, e.g., a smartphone that is running a messaging application, and receiving responsive messages from the user in return.

Data processing apparatus for implementing machine learning models can also include, for example, special-purpose hardware accelerator units for processing common and compute-intensive parts of machine learning training or production, i.e., inference, workloads.

Machine learning models can be implemented and deployed using a machine learning framework, e.g., a TensorFlow framework, or a Jax framework.

Embodiments of the subject matter described in this specification can be implemented in a computing system that includes a back-end component, e.g., as a data server, or that includes a middleware component, e.g., an application server, or that includes a front-end component, e.g., a client computer having a graphical user interface, a web browser, or an app through which a user can interact with an implementation of the subject matter described in this specification, or any combination of one or more such back-end, middleware, or front-end components. The components of the system can be interconnected by any form or medium of digital data communication, e.g., a communication network. Examples of communication networks include a local area network (LAN) and a wide area network (WAN), e.g., the Internet.

The computing system can include clients and servers. A client and server are generally remote from each other and typically interact through a communication network. The relationship of client and server arises by virtue of computer programs running on the respective computers and having a client-server relationship to each other. In some embodiments, a server transmits data, e.g., an HTML page, to a user device, e.g., for purposes of displaying data to and receiving user input from a user interacting with the device, which acts as a client. Data generated at the user device, e.g., a result of the user interaction, can be received at the server from the device.

While this specification contains many specific implementation details, these should not be construed as limitations on the scope of any invention or on the scope of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially be claimed as such, one or more features from a claimed combination can in some cases be excised from the combination, and the claimed combination may be directed to a subcombination or variation of a subcombination.

Similarly, while operations are depicted in the drawings and recited in the claims in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In some cases, multitasking and parallel processing may be advantageous.

Further aspects of the invention are defined in the following clauses:
1. A computer implemented method comprising:
   presenting, on a display of a user device and as part of a graphical user interface: (i) a graphical representation of a chemical structure of a current molecule, and (ii) a respective current value, for the current molecule, of each molecule property in a set of molecule properties, using a set of molecule data;
   dynamically updating the display of the user device as a user interacts with the graphical user interface, comprising, at each of a plurality of update iterations:
      initiating the update iteration in response to determining that a user of the user device has interacted with the graphical user interface to provide a user input that defines a request to modify the chemical structure of the molecule being presented on the display of the user device;
      generating, based on the user input, a set of updated molecule data defining an updated molecule having a different chemical structure than the current molecule;
      automatically determining a respective updated value, for the updated molecule, of each molecule property in the set of molecule properties using the set of updated molecule data; and
      updating the display of the user device to present: (i) a graphical representation of the updated molecule, and (ii) the respective updated value, for the updated molecule, of each molecule property in the set of molecule properties.
2. The method of clause 1, wherein automatically determining a respective updated value, for the updated molecule, of each molecule property in the set of molecule properties comprises, for each of one or more molecule properties:
   determining whether a precomputed value of the molecule property of the updated molecule is available in a molecule property database.
3. The method of clause 2, further comprising, in response to determining that the precomputed value of the molecule property of the updated molecule is available in the molecule property database:
   determining the updated value of the molecule property of the updated molecule by accessing the precomputed value of the molecule property of the updated molecule from the molecule property database.
4. The method of clause 2, further comprising, in response to determining that the precomputed value of the molecule property of the updated molecule is not available in the molecule property database:
   processing a model input based on the set of updated molecule data using a molecule property prediction machine learning model, in accordance with values of a set of machine learning model parameters, to generate the updated value of the molecule property of the updated molecule.
5. The method of clause 4, further comprising, after generating the updated value of the molecule property of the updated molecule:
   storing: (i) the set of updated molecule data, and (ii) the updated value of the molecule property of the updated molecule, in a molecule property database.
6. The method of any one of clauses 2-5, wherein determining whether the precomputed value of the molecule property of the updated molecule is available in the molecule property database comprises:
   determining whether a molecule identifier that identifies the updated molecule is indexed in the molecule property database.
7. The method of any one of clauses 1-6, wherein the set of molecule properties comprises one or more of: absorption, distribution, metabolism, excretion, and toxicity properties.
8. The method of any one of clauses 1-7, wherein determining that the user of the user device has interacted with the graphical user interface to provide the user input that defines the request to modify the chemical structure of the molecule being presented on the display of the user device comprises:
   determining that the user has drawn an addition to or a deletion from the chemical structure of the molecule by way of the graphical user interface.
9. The method of any one of clauses 1-7, wherein determining that the user of the user device has interacted with the graphical user interface to provide the user input that defines the request to modify the chemical structure of the molecule being presented on the display of the user device comprises:
   determining that the user has selected and rotated a portion of the molecule around a rotatable bond by way of the graphical user interface.
10. The method of any one of clauses 1-7, wherein determining that the user of the user device has interacted with the graphical user interface to provide the user input that defines the request to modify the chemical structure of the molecule being presented on the display of the user device comprises:
   determining that the user has input one or more alphanumerical characters specifying a modification to the chemical structure.
11. The method of any one of clauses 1-7, wherein determining that the user of the user device has interacted with the graphical user interface to provide the user input that defines the request to modify the chemical structure of the molecule being presented on the display of the user device comprises:
   receiving, from the user and by way of the graphical user interface, a selection of: (i) a portion of the molecule to be replaced, and (ii) a molecule fragment to be substituted into the portion of the molecule to be replaced, wherein the molecule fragment is included in a predefined collection of molecule fragments that are selectable to the user by way of the graphical user interface.
12. The method of any one of clauses 1-7, wherein determining that the user of the user device has interacted with the graphical user interface to provide the user input that defines the request to modify the chemical structure of the molecule being presented on the display of the user device comprises:
   determining that the user has interacted with the graphical user interface to select a portion of the molecule to be replaced; and
   wherein generating, based on the user input, the set of updated molecule data defining an updated molecule having a different chemical structure than the current molecule comprises:
      generating the set of updated molecule data defining the updated molecule as an output of a generative machine learning model that is conditioned on data identifying the portion of the molecule to be replaced.
13. The method of any one of clauses 1-12, wherein the display of the user device is updated to present: (i) the graphical representation of the updated molecule, and (ii) the respective updated value, for the updated molecule, of each molecule property in the set of molecule properties, within one second of determining that the user of the user device has interacted with the graphical user interface.
14. The method of any one of clauses 1-13, wherein the graphical representation of the chemical structure comprises a two-dimensional (2D) molecule representation comprising one or more of a skeletal, Lewis, or ball-and-stick molecule representation.
15. The method of any one of clauses 1-13, wherein the graphical representation of the chemical structure comprises a three-dimensional (3D) molecule representation.
16. The method of clause 15, wherein the graphical user interface enables the user to dynamically reorient the 3D molecule representation to provide different views of the chemical structure.
17. The method of any one of clauses 15-16, wherein the graphical user interface is configured to reorient the 3D molecule representation in response to a user selecting a portion of the molecule and dragging the portion along one or more directions.
18. The method of any one of clauses 1-17, wherein the graphical representation is configurable by a user, and wherein configuring the graphical representation comprises one or more of:
   i) specifying one or more water molecules be mapped and displayed to corresponding positions with respect to the graphical representation of the chemical structure;
   ii) specifying a showing of electrostatic clashes; and
   iii) determining one or more colors of the rendered chemical structure, where the one or more colors correspond with respective portions of the rendered structure; and
   determining an opacity of the rendered chemical structure.
19. The method of any one of clauses 1-18, further comprising, prior to presenting, on a display of a user device and as part of a graphical user interface: (i) a graphical representation of a chemical structure of a current molecule, and (ii) a respective current value, for the current molecule, of each molecule property in a set of molecule properties, using a set of molecule data:
   receiving a user input that selects the current molecule from a molecule database, wherein each molecule in the molecule database is associated with a respective set of molecule data defining a chemical structure of the molecule; and
   accessing the set of molecule data for the current molecule from the molecule database.
20. A method of performing drug discovery using the method of any one of clauses 1-19, wherein performing the drug discovery comprises one or both of:
   i) identifying an agonist or antagonist of a receptor or enzyme, and
   ii) performing hit optimization of an agonist or antagonist of a receptor or enzyme.
21. The method of any one of clauses 1-20, further comprising:
   determining a plurality of the updated molecules;
   receiving from the graphical user interface, for each of one or more molecule properties, data that: (i) partitions a range of possible values of the molecule property into a plurality of intervals,
   and (ii) associates each of the plurality of intervals with a respective desirability rating from a set of desirability ratings;
      determining a respective loss score for each of the updated molecules, comprising:
         determining, for each of the one or more molecule properties, a respective value of the molecule property for the updated molecule;
         determining, for each of the one or more molecule properties, a respective property-specific loss based at least in part on a desirability rating of an interval that includes the value of the molecule property for the updated molecule; and
         generating the loss score for the updated molecule by combining the respective property-specific losses for each of the one or more molecule properties; and
      providing to the user a representation of the loss scores for the updated molecules.
22. A system comprising one or more computers and one or more storage devices storing instructions that are operable, when executed by the one or more computers, to cause the one or more computers to perform the method of any one of clauses 1-21.
23. A computer storage medium encoded with a computer program, the program comprising instructions that are operable, when executed by data processing apparatus, to cause the data processing apparatus to perform the method of any one of clauses 1-21.
24. A computer implemented method comprising:
   receiving data identifying a collection of molecules;
   receiving, for each of one or more molecule properties, data that: (i) partitions a range of possible values of the molecule property into a plurality of intervals, and (ii) associates each of the plurality of intervals with a respective desirability rating from a set of desirability ratings;
   determining a respective loss score for each molecule in the collection of molecules, comprising, for each molecule in the collection of molecules:
      determining, for each of the one or more molecule properties, a respective value of the molecule property for the molecule;
      determining, for each of the one or more molecule properties, a respective property-specific loss based at least in part on a desirability rating of an interval that includes the value of the molecule property for the molecule; and
      generating the loss score for the molecule by combining the respective property-specific losses for each of the one or more molecule properties; and
   providing the loss scores for the molecules in the collection of molecules.
25. The method of clause 24, wherein the set of desirability ratings comprises at least three desirability ratings.
26. The method of any one of clauses 24-25, wherein for each of the one or more molecule properties, receiving data that: (i) partitions a range of possible values of the molecule property into a plurality of intervals, and (ii) associates each of the plurality of intervals with a respective desirability rating from a set of desirability ratings comprises:
   receiving, from a user, and in particular by way of a graphical user interface, a user input that identifies respective endpoints of each of the plurality of intervals.
27. The method of clause 26, wherein receiving, from the user and by way of the graphical user interface, the user input that identifies respective endpoints of each of the plurality of intervals comprises:
   presenting, to the user and by way of the graphical user interface, a slider that includes: (i) a track, and (ii) a plurality of indicator elements defining endpoints of the plurality of intervals; and
   receiving, from the user and by way of the graphical user interface, one or more user inputs that define adjustments to respective positions of one or more of the plurality of indicator elements.
28. The method of clause 27, wherein receiving, from the user and by way of the graphical user interface, one or more user inputs that define adjustments to respective positions of one or more of the plurality of indicator elements comprises:
   receiving one or more user inputs indicating that the user has dragged a position of an indicator element along the track to adjust the position of the indicator element.
29. The method of any one of clauses 27-28, wherein the track represents the range of possible values of the molecule property; and
   wherein presenting, to the user and by way of the graphical user interface, the slider that includes: (i) the track, and (ii) the plurality of indicator elements defining endpoints of the plurality of intervals comprises, for each of the plurality of intervals:
   displaying the interval in a color associated with the desirability rating associated with the interval.
30. The method of clause 24-29, wherein for one or more of the molecule properties, determining the value of the molecule property for the molecule comprises:
   processing a model input that characterizes the molecule using a property prediction machine learning model, in accordance with values of a set of machine learning model parameters, to generate the value of the molecule property for the molecule.
31. The method of any one of clauses 24-30, wherein each desirability rating in the set of desirability ratings is associated with a respective penalty function; and
   wherein determining, for each of the one or more molecule properties, a respective property-specific loss based at least in part on the desirability rating of the interval that includes the value of the molecule property for the molecule further comprises, for each molecule property:
   determining the property-specific loss for the molecule property by applying the penalty function associated with the interval that includes the value of the molecule property to the value of the molecule property.
32. The method of clause 31, wherein the desirability ratings in the set of desirability ratings are associated with a ranking;
   wherein the penalty function for a desirability rating ranked first in the set of desirability ratings is a constant zero function;
   wherein the penalty function for a desirability rating ranked second in the set of desirability ratings comprises a linear function of an input molecule property value; and
   wherein the penalty function for a desirability rating ranked third in the set of desirability ratings comprises a quadratic function of an input molecule property value.
33. The method of any one of clauses 24-32, wherein generating the loss score for the molecule by combining the respective property-specific losses for each of the one or more molecule properties comprises:
   scaling each property-specific loss by an importance factor corresponding to the property-specific loss.
34. The method of any one of clauses 24-33, wherein receiving, for each of one or more molecule properties, data that: (i) partitions a range of possible values of the molecule property into a plurality of intervals, and (ii) associates each of the plurality of intervals with a respective desirability rating from a set of desirability ratings, comprises:
   receiving data identifying a target protein;
   identifying a collection of proteins that each satisfy one or more similarity criteria with respect to the target protein;
   identifying a collection of ligands that each bind to a respective protein in the collection of proteins; and
   determining, for one or more of the molecule properties, the plurality of intervals and the respective desirability rating of each interval based on a distribution of values of the molecule property over the collection of ligands.
35. The method of any one of clauses 24-34, wherein providing the loss scores for the molecules in the collection of molecules comprises:
   determining a ranking of the molecules in the collection of molecules based on the loss scores.
36. The method of any one of clauses 24-34, wherein providing the loss scores for the molecules in the collection of molecules comprises:
   identifying a set of candidate molecules from the collection of molecules for binding with a target molecule based at least in part on the loss scores; and
   evaluating a measure of binding affinity for each molecule in the collection of molecules for a target protein.
37. The method of clause 36, further comprising:
   ranking the molecules in accordance with a predicted binding affinity; and
   selecting one or more of the candidate molecules for physical synthesis based at least in part on the respective binding affinity.
38. The method of any one of clauses 24-37, wherein receiving data identifying a collection of molecules comprises receiving data comprising a collection of custom ligands or a collection of ligands in a library.
39. A system comprising one or more computers and one or more storage devices storing instructions that are operable, when executed by the one or more computers, to cause the one or more computers to perform the method of any one of clauses 24-38.
40. A computer storage medium encoded with a computer program, the program comprising instructions that are operable, when executed by data processing apparatus, to cause the data processing apparatus to perform the method of any one of clauses 24-38.

What is claimed is:

## Claims

1. A computer implemented method comprising:
presenting, on a display of a user device and as part of a graphical user interface: (i) a graphical representation of a chemical structure of a current molecule, and (ii) a respective current value, for the current molecule, of each molecule property in a set of molecule properties, using a set of molecule data;
dynamically updating the display of the user device as a user interacts with the graphical user interface, comprising, at each of a plurality of update iterations:
initiating the update iteration in response to determining that a user of the user device has interacted with the graphical user interface to provide a user input that defines a request to modify the chemical structure of the molecule being presented on the display of the user device;
generating, based on the user input, a set of updated molecule data defining an updated molecule having a different chemical structure than the current molecule;
automatically determining a respective updated value, for the updated molecule, of each molecule property in the set of molecule properties using the set of updated molecule data; and
updating the display of the user device to present: (i) a graphical representation of the updated molecule, and (ii) the respective updated value, for the updated molecule, of each molecule property in the set of molecule properties.

2. The method of claim 1, wherein automatically determining a respective updated value, for the updated molecule, of each molecule property in the set of molecule properties comprises, for each of one or more molecule properties:
determining whether a precomputed value of the molecule property of the updated molecule is available in a molecule property database.

3. The method of claim 2, further comprising, in response to determining that the precomputed value of the molecule property of the updated molecule is available in the molecule property database:
determining the updated value of the molecule property of the updated molecule by accessing the precomputed value of the molecule property of the updated molecule from the molecule property database.

4. The method of claim 2, further comprising, in response to determining that the precomputed value of the molecule property of the updated molecule is not available in the molecule property database:
processing a model input based on the set of updated molecule data using a molecule property prediction machine learning model, in accordance with values of a set of machine learning model parameters, to generate the updated value of the molecule property of the updated molecule.

5. The method of claim 4, further comprising, after generating the updated value of the molecule property of the updated molecule:
storing: (i) the set of updated molecule data, and (ii) the updated value of the molecule property of the updated molecule, in a molecule property database.

6. The method of any one of claims 2-5, wherein determining whether the precomputed value of the molecule property of the updated molecule is available in the molecule property database comprises:
determining whether a molecule identifier that identifies the updated molecule is indexed in the molecule property database.

7. The method of any one of claims 1-6, wherein the set of molecule properties comprises one or more of: absorption, distribution, metabolism, excretion, and toxicity properties.

8. The method of any one of claims 1-7, wherein determining that the user of the user device has interacted with the graphical user interface to provide the user input that defines the request to modify the chemical structure of the molecule being presented on the display of the user device comprises:
determining that the user has drawn an addition to or a deletion from the chemical structure of the molecule by way of the graphical user interface; or
wherein determining that the user of the user device has interacted with the graphical user interface to provide the user input that defines the request to modify the chemical structure of the molecule being presented on the display of the user device comprises:
determining that the user has selected and rotated a portion of the molecule around a rotatable bond by way of the graphical user interface; or
wherein determining that the user of the user device has interacted with the graphical user interface to provide the user input that defines the request to modify the chemical structure of the molecule being presented on the display of the user device comprises:
determining that the user has input one or more alphanumerical characters specifying a modification to the chemical structure; or
wherein determining that the user of the user device has interacted with the graphical user interface to provide the user input that defines the request to modify the chemical structure of the molecule being presented on the display of the user device comprises:
receiving, from the user and by way of the graphical user interface, a selection of: (i) a portion of the molecule to be replaced, and (ii) a molecule fragment to be substituted into the portion of the molecule to be replaced, wherein the molecule fragment is included in a predefined collection of molecule fragments that are selectable to the user by way of the graphical user interface; or
wherein determining that the user of the user device has interacted with the graphical user interface to provide the user input that defines the request to modify the chemical structure of the molecule being presented on the display of the user device comprises:
determining that the user has interacted with the graphical user interface to select a portion of the molecule to be replaced; and
wherein generating, based on the user input, the set of updated molecule data defining an updated molecule having a different chemical structure than the current molecule comprises:
generating the set of updated molecule data defining the updated molecule as an output of a generative machine learning model that is conditioned on data identifying the portion of the molecule to be replaced.

9. The method of any one of claims 1-8, wherein the graphical representation is configurable by a user, and wherein configuring the graphical representation comprises one or more of:
i) specifying one or more water molecules be mapped and displayed to corresponding positions with respect to the graphical representation of the chemical structure;
ii) specifying a showing of electrostatic clashes; and
iii) determining one or more colors of the rendered chemical structure, where the one or more colors correspond with respective portions of the rendered structure; and
determining an opacity of the rendered chemical structure.

10. The method of any one of claims 1-9, further comprising, prior to presenting, on a display of a user device and as part of a graphical user interface: (i) a graphical representation of a chemical structure of a current molecule, and (ii) a respective current value, for the current molecule, of each molecule property in a set of molecule properties, using a set of molecule data:
receiving a user input that selects the current molecule from a molecule database, wherein each molecule in the molecule database is associated with a respective set of molecule data defining a chemical structure of the molecule; and
accessing the set of molecule data for the current molecule from the molecule database.

11. A method of performing drug discovery using the method of any one of claims 1-10, wherein performing the drug discovery comprises one or both of:
i) identifying an agonist or antagonist of a receptor or enzyme, and
ii) performing hit optimization of an agonist or antagonist of a receptor or enzyme.

12. The method of any one of claims 1-11, further comprising:
determining a plurality of the updated molecules;
receiving from the graphical user interface, for each of one or more molecule properties, data that: (i) partitions a range of possible values of the molecule property into a plurality of intervals, and (ii) associates each of the plurality of intervals with a respective desirability rating from a set of desirability ratings;
determining a respective loss score for each of the updated molecules, comprising:
determining, for each of the one or more molecule properties, a respective value of the molecule property for the updated molecule;
determining, for each of the one or more molecule properties, a respective property-specific loss based at least in part on a desirability rating of an interval that includes the value of the molecule property for the updated molecule; and
generating the loss score for the updated molecule by combining the respective property-specific losses for each of the one or more molecule properties; and
providing to the user a representation of the loss scores for the updated molecules.

13. A system comprising one or more computers and one or more storage devices storing instructions that are operable, when executed by the one or more computers, to cause the one or more computers to perform the method of any one of claims 1-12.

14. A computer storage medium encoded with a computer program, the program comprising instructions that are operable, when executed by data processing apparatus, to cause the data processing apparatus to perform the method of any one of claims 1-12.

15. A computer implemented method comprising:
receiving data identifying a collection of molecules;
receiving, for each of one or more molecule properties, data that: (i) partitions a range of possible values of the molecule property into a plurality of intervals, and (ii) associates each of the plurality of intervals with a respective desirability rating from a set of desirability ratings;
determining a respective loss score for each molecule in the collection of molecules, comprising, for each molecule in the collection of molecules:
determining, for each of the one or more molecule properties, a respective value of the molecule property for the molecule;
determining, for each of the one or more molecule properties, a respective property-specific loss based at least in part on a desirability rating of an interval that includes the value of the molecule property for the molecule; and
generating the loss score for the molecule by combining the respective property-specific losses for each of the one or more molecule properties; and
providing the loss scores for the molecules in the collection of molecules.
